# EUROPEAN PATENT APPLICATION

(11) **EP 4 029 564 A1**
(43) Date of publication of application: **20.07.2022**
(21) Application number: 21218481.6
(22) Date of filing: 31.12.2021
(51) Int. Cl.: A61N 5/10

(54) **RADIATION THERAPY PLANNING SYSTEM, METHOD AND COMPUTER PROGRAM FOR PLANNING A RADIATION THERAPY PROCEDURE**

(30) Priority: 14.01.2021 EP 21151480
(71) Applicant: Koninklijke Philips N.V., 5656 AG Eindhoven (NL)
(72) Inventor: ANDERSSON, Joel, Arvid, Emanuel, 5656 AG Eindhoven (NL)
(74) Representative: Philips Intellectual Property & Standards

(57) **Abstract**

The invention relates to a radiation therapy planning system 8 for planning a radiation therapy procedure. A quality reducing unit 11 reduces the quality of a higher quality ODM 51 to create a quality reduced ODM 52, and a treatment plan generating unit 12 generates a treatment plan by performing an optimization that includes a first optimization that independently optimizes for each leaf-pair 41 of an MLC 4 one or more leaf configurations such that the cumulative fluence of a radiation beam 3 shaped by the leaf configurations of the leaf-pair approximates a corresponding portion of the quality reduced ODM. A higher quality optimization procedure is later performed on the higher quality ODM based on a local search algorithm that is initialized with the generated treatment plan to generate a higher quality treatment plan. The invention replaces a critical step in the planning process with a deterministic, global algorithm.

## Description

### FIELD OF THE INVENTION

The invention relates to a radiation therapy planning system, method and computer program for planning a radiation therapy procedure. The invention relates further to a radiation therapy system comprising the radiation therapy planning system.

### BACKGROUND OF THE INVENTION

Typical radiation therapy systems comprise a radiation source, which is configured to emit a radiation beam for treating a subject, and a multi-leaf collimator (MLC), which comprises multiple leaf-pairs of movable leaves for forming an aperture of the MLC such that the radiation beam is shaped by the aperture. A rotating unit rotates the radiation source and the MLC around the subject in order to apply the shaped radiation beam to the subject from multiple positions. In order to perform a radiation therapy procedure, the radiation source, the MLC and the rotating unit are controlled by a controller in accordance with a treatment plan comprising treatment parameters like, for instance, for different positions of the radiation source relative to the subject different configurations of the leaf-pairs of the MLC. The treatment plan is generated by using an optimization algorithm, which tries to minimize deviations between a simulated dose distribution within the subject, which is simulated based on the treatment parameters to be determined, and a desired dose distribution within the subject.

This known generation of the treatment plan has the drawback that for certain types of radiation therapy procedures, such as volumetric modulated arc therapy (VMAT), the optimization algorithm might not be able to find the optimal treatment parameters for a given radiation delivery time (see J. Unkelbach et al., "Optimization approaches to volumetric modulated arc therapy planning", Med. Phys., Vol. 42, No. 3, pages 1367 to 1377 (2015)). This is due to the fact that the treatment planning problem for these radiation therapy procedures is a fundamentally non-convex optimization problem, preventing the optimal solution to be found using local search approaches, while at the same time having too large dimension to be addressed with typical global optimization approaches. Instead, existing software solutions rely on stochastic or heuristic initialization approaches, neither of which can guarantee that an optimal solution - in any sense - is found in a deterministic amount of time.

### SUMMARY OF THE INVENTION

It is an object of the present invention to provide a radiation therapy planning system, method and computer program, which allow for an improved planning of a radiation therapy procedure. It is a further object of the present invention to provide a radiation therapy system comprising the radiation therapy planning system.

In a first aspect of the present invention, a radiation therapy planning system for planning a radiation therapy procedure is presented, wherein the radiation therapy procedure includes rotating a radiation source, which is configured to emit a radiation beam for treating a subject, and an MLC, which comprises multiple leaf-pairs of movable leaves for forming an aperture of the MLC such that the radiation beam is shaped by the aperture, along an arc around the subject in order to apply the shaped radiation beam to the subject from at least one position on the arc, wherein the radiation therapy planning system comprises:
- a quality reducing unit configured to reduce the quality of a higher quality opening density matrix (ODM) comprising bixels, which provide target beamlet fluences, to create a quality reduced ODM, and
- a treatment plan generating unit configured to generate a treatment plan by performing an optimization that includes a first optimization that independently optimizes for each leaf-pair of the MLC one or more leaf configurations such that the cumulative fluence of the radiation beam shaped by the one or more leaf configurations of the leaf-pair approximates a corresponding portion of the quality reduced ODM,
wherein the treatment plan generating unit is further configured to perform a higher quality optimization procedure on the higher quality ODM based on a local search algorithm that is initialized with the generated treatment plan in order to generate a higher quality treatment plan.

The invention aims at providing a remedy to the above-outlined optimization issues associated with the known radiation therapy planning approaches. By reducing the quality of a higher quality ODM to create a quality reduced ODM and by generating a treatment plan by performing an optimization that includes a first optimization that independently optimizes for each leaf-pair of the MLC one or more leaf configurations such that the cumulative fluence of the radiation beam shaped by the one or more leaf configurations of the leaf-pair approximates a corresponding portion of the quality reduced ODM, a global solution to an approximation of the treatment planning problem may be found in a deterministic amount of computation time and with a deterministic amount of memory use. This approximate solution, i.e., the generated treatment plan, can then be used to initialize a local search algorithm for a more accurate problem formulation that better captures all machine constraints in order to generate a higher quality treatment plan for the higher quality ODM. Thus, the invention replaces a critical step in the traditional intensity modulated radiation therapy (IMRT) or VMAT planning process, which is currently treated heuristically or with algorithms without convergence guarantees, with a deterministic, global algorithm.

The invention assumes that one or more ODMs (also known as "fluence maps" or "intensity maps") for a treatment plan have already been found using a fluence map optimization (FMO) technique. Since the FMO problem can be addressed with convex optimization techniques time (see J. Unkelbach et al. (2015), ibid), a global solution to the FMO problem can be found using gradient-based local search approaches.

It is preferred that the quality reducing unit is configured to reduce the higher quality ODM in spatial resolution and/or in the number of different possible beamlet fluence values. The quality of the approximate solution deterministically depends on how well the approximation of the treatment planning problem resembles the original problem, which is known before the global optimization starts. By reducing the higher quality ODM in spatial resolution and/or in the number of different possible beamlet fluence values, a desired trade-off between the quality of the approximation of the treatment planning problem and the resources (computation time, memory use) required for finding the approximate solution can be selected.

It is further preferred that the treatment plan generating unit comprises a dynamic programming (DP) unit configured to perform the optimization for each leaf-pair of the MLC using a DP forward recursion. DP is an algorithmic paradigm that solves a given complex problem by breaking it into subproblems and that stores the results of the subproblems to avoid computing the same results again. As such, DP is very well suited for optimizing one or more leaf configurations for each leaf-pair of the MLC with a rather small amount of memory use. The number of the one or more leaf configurations for a given leaf-pair may typically not be known beforehand. The DP forward recursion may thus be run, for instance, until the cumulative fluence of the radiation beam shaped by the one or more leaf configurations of the leaf-pair perfectly corresponds to the corresponding portion of the quality reduced ODM or until a desired error criterion is met.

It is preferred that the state space of the DP forward recursion is given by the number of possible cumulative beamlet fluence values at each step of the DP forward recursion, wherein the DP unit is configured to cap the state space for each bixel to the target beamlet fluence for the bixel. Preferably, the DP algorithm globally solves an optimal control problem with a state space given by the cumulative fluence of the shaped radiation beam, with the goal to minimize the deviation, in L-1 norm, from the corresponding portion of the quality reduced ODM (target fluence). The computational complexity of this step is exponential in the size of the state space, i.e., the number of possible cumulative fluence configurations considered at any step of the algorithm. By capping the state space for each bixel in the quality reduced ODM at the target fluence, the size of the state space can be vastly decreased. Since the fluence contribution from each step is always positive, cost contributions in excess of the target fluence can be handled as a stage cost in the DP formulation, without affecting the optimality of the solution. This idea to cap the state space, in combination with a highly optimized implementation in parallelized integer arithmetic, allows solutions to realistic sized ODMs to be calculated in fractions of a second.

It is further preferred that the treatment plan generating unit comprises a same number determining unit configured to determine a same number of the one or more leaf configurations to be used for each leaf-pair of the MLC to shape the radiation beam such that the cumulative fluence of the shaped radiation beam approximates the quality reduced ODM based on the independently optimized one or more leaf configurations for each leaf pair of the MLC. Preferably, by performing the DP forward recursion for each leaf-pair independently and stopping after each additional leaf configuration to evaluate the result so far, it is possible for each leaf-pair to obtain a trade-off curve between the number of the one or more leaf configurations and the deviation from the target fluence. For instance, for a given leaf-pair a total of 6 leaf configurations may be required to perfectly reconstruct the corresponding portion of the quality reduced ODM while for other leaf-pairs only 3, 4 or 5 or even a smaller number of leaf configurations may be necessary. Based on these trade-off curves the same number of the one or more leaf configurations to be used for each leaf-pair of the MLC may be determined such that the apertures of the MLC formed by the determined same number of the one or more leaf configurations of each leaf-pair of the MLC may allow for a perfect reconstruction of the quality reduced ODM or for a reconstruction of the quality reduced ODM such that a desired error criterion is met.

It is preferred that the optimization includes a second optimization that optimizes for each leaf-pair of the MLC the determined same number of the one or more leaf configurations, wherein a regularization is used such that a deviation of the leaf configurations of a currently optimized leaf-pair from the leaf configurations of a neighboring, previously optimized leaf-pair is reduced. The first optimization generally does not have a unique solution. For instance, other solutions with the same fitting error may be obtained by permutating the order of the leaf configurations, or by exploiting the fact that most leaf-pairs may require less than the determined same number of the one or more leaf configurations to reconstruct the corresponding portion of the reduced quality ODM. These degrees of freedom can be exploited in the second optimization, e.g., in a second application of the DP algorithm, this time adding a secondary objective to the optimization formulation (DP formulation) that reduces the deviation between neighboring, optimized leaf-pairs without sacrificing the fitting objective.

It is further preferred that the second optimization includes an ordering heuristic such that the optimizing starts with a leaf-pair of the MLC that required a larger number of leaf configurations in the first optimization. The ordering heuristic can be determined e.g. based on the trade-off curves between the number of the one or more leaf configurations and the deviation from the target fluence described above. The ordering heuristic may employ a greedy strategy. Such a strategy does not usually produce an optimal solution, but nonetheless, a greedy heuristic may yield locally optimal solutions that approximate a globally optimal solution in a reasonable amount of time.

It is preferred that the second optimization includes a centering of closed leaf configurations such as to smooth the transitions between neighboring leaf-pairs of the MLC. While the use of the ordering heuristic and the centering of the closed leaf configurations may not guarantee optimality in terms of the apertures of the MLC, it attempts to get apertures that are reasonable smooth and, thus, consistent with treatment plan quality objectives.

It is further preferred that the radiation therapy procedure is a step-and-shot IMRT procedure, wherein the apertures of the MLC formed by the determined same number of the one or more leaf configurations of each leaf-pair of the MLC to be used to sequentially shape the radiation beam emitted by the radiation source from a single position on the arc.

It is preferred that the radiation therapy procedure is a VMAT procedure, wherein each of the apertures of the MLC formed by the determined same number of the one or more leaf configurations of each leaf-pair of the MLC is to be used to shape the radiation beam emitted by the radiation source from a different position on a segment of the arc.

In a VMAT procedure, the radiation source is typically moving along an arc in a continuous manner, emitting radiation all the time. For the final simulated dose, this dose is approximated by a finite sum of discrete dose contributions from different angular positions along the arc, where the angular sampling is rather fine (e.g., every 4 degrees, or every 2 degrees), where each angular position occurs only once. The dose contribution for each of these angular positions is then characterized by an individual MLC configuration, where each of the dose contributions is weighted by the scalar fluence intensity that the radiation beam has at that angular position. The ODMs that are given as input to the VMAT procedure, however, are generally sampled at a much coarser sampling (e.g. every 24 degrees, or every 52 degrees), such that a corresponding position of the arc is typically called a "segment". Hence, these segments are representing the combined contribution of several MLC configurations, but the entire arc may still consist of several of those segments.

It is further preferred that the treatment plan generating unit is configured to perform the optimization for each of two or more segments on the arc, wherein a separate ODM is used for each segment, wherein a consistency constraint is used for consecutive segments such as to smooth the transition between the apertures of the MLC between the consecutive segments.

Two consecutive segments of the arc are not fully independent, since the last MLC aperture of the first segment and the first MLC aperture of the second segment are also consecutive as MLC apertures of the final simulated dose. As such, they are optimized to allow that the leaves can move from the first MLC aperture to the second MLC aperture within the time given for the radiation source to move the distance of 4 (resp. 2) degrees. This leads to the requirement of a consistency between the solutions to different consecutive segments.

Finally, in the context of a VMAT procedure, the above ordering heuristic typically leads to the general behavior within one segment, e.g., that there is a global movement direction of the leaf-pairs (either left-to-right, or right-to-left). This observation yields a sorting heuristic on segment level for consecutive segments.

In a further aspect of the present invention, a radiation therapy system is presented, comprising:
- a radiation source configured to emit a radiation beam for treating a subject,
- an MLC comprising multiple leaf-pairs of movable leaves for forming an aperture of the MLC such that the radiation beam is shaped by the aperture,
- a rotating unit for rotating the radiation source and the MLC along an arc around the subject in order to apply the shaped radiation beam to the subject from at least one position on the arc,
- a radiation therapy planning system for planning a radiation therapy procedure as defined in claim 1,
- a controller for controlling the radiation source, the MLC and the rotating unit in accordance with the planned radiation therapy procedure.

In another aspect of the present invention, a radiation therapy planning method for planning a radiation therapy procedure is presented, wherein the radiation therapy procedure includes rotating a radiation source, which is configured to emit a radiation beam for treating a subject, and a multi-leaf collimator, MLC, which comprises multiple leaf-pairs of movable leaves for forming an aperture of the MLC such that the radiation beam is shaped by the aperture, along an arc around the subject in order to apply the shaped radiation beam to the subject from at least one position on the arc, wherein the radiation therapy planning method comprises:
- reducing, by a quality reducing unit, the quality of a higher quality opening density matrix, ODM, comprising bixels, which provide target beamlet fluences, to create a quality reduced ODM,
- generating, by a treatment plan generating unit, a treatment plan by performing an optimization that includes a first optimization that independently optimizes for each leaf-pair of the MLC one or more leaf configurations such that the cumulative fluence of the radiation beam shaped by the one or more leaf configurations of the leaf-pair approximates a corresponding portion of the quality reduced ODM, and
- performing, by the treatment plan generating unit, a higher quality optimization procedure on the higher quality ODM based on a local search algorithm that is initialized with the generated treatment plan in order to generate a higher quality treatment plan.

In a further aspect of the present invention, a radiation therapy planning computer program for planning a radiation therapy procedure is presented, the computer program comprising program code means for causing a radiation therapy planning system as defined in claim 1 to carry out the steps of the radiation therapy planning method as defined in claim 14, when the computer program is run on a computer controlling the radiation therapy planning system.

It shall be understood that the radiation therapy planning system of claim 1, the radiation therapy system of claim 12, the radiation therapy planning method of claim 13, and the radiation therapy planning computer program of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a radiation therapy system,
Fig. 2 shows schematically and exemplarily a MLC of use in the radiation therapy system of Fig. 1,
Fig. 3 shows schematically and exemplarily an embodiment of a radiation therapy planning system for planning a radiation therapy procedure,
Fig. 4 shows schematically and exemplarily a higher quality ODM and a quality reduced ODM,
Fig. 5 shows schematically and exemplarily trade-off curves between the number of the one or more leaf configurations and the deviation from the target fluence,
Fig. 6 shows schematically and exemplarily 6 optimized apertures of the MLC, and
Fig. 7 shows a flowchart exemplarily illustrating an embodiment of a radiation therapy planning method for planning a radiation therapy procedure.

### DETAILED DESCRIPTION OF EMBODIMENTS

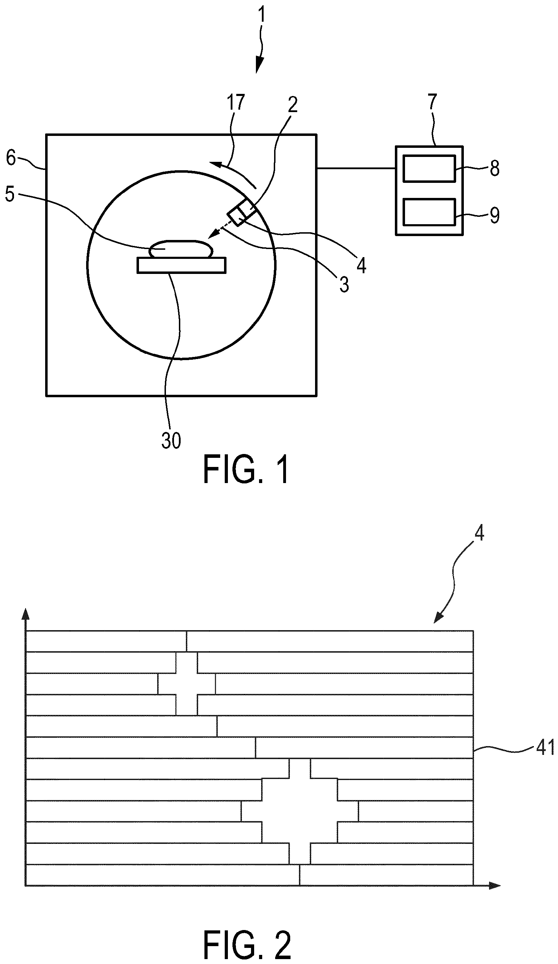

Fig. 1 shows schematically and exemplarily an embodiment of a radiation therapy system being a VMAT system. The VMAT system 1 comprises a radiation source 2 configured to emit a radiation beam 3 for treating a subject 5 and an MLC 4 for shaping the radiation beam 3. In this embodiment, the subject 5 is a human patient lying on a support means 30 like a patient table. In another embodiment the subject can also be an animal. The radiation source 2 and the MLC 4 are attached to a gantry 6 configured to rotate the radiation source 2 and the MLC 4 along an arc around the subject 5 in the direction indicated by the arrow 17, in order to apply the shaped radiation beam 3 to the subject 5 from at least one position on the arc.

An MLC 4 for use in the radiation therapy system 1 is schematically and exemplarily shown in Fig. 2. It comprises multiple leaf-pairs 41 of movable leaves for forming an aperture of the MLC 4 such that the radiation beam 3 is shaped by the aperture. In this example, the aperture has the shape of two openings, a smaller one in the upper part of the MLC 4 and a larger one in the lower part of the MLC 4.

With returning reference to Fig. 1, the VMAT system 1 further comprises a control system 7 with a radiation therapy planning system 8 for planning a radiation therapy procedure to be carried out by the VMAT system 1 and a controller 9 for controlling at least the radiation source 2, the MLC 4 and the gantry 6 in accordance with the planned radiation therapy procedure. The controller 9 can also be adapted to control further parts of the VMAT system 1 like the support means 30.

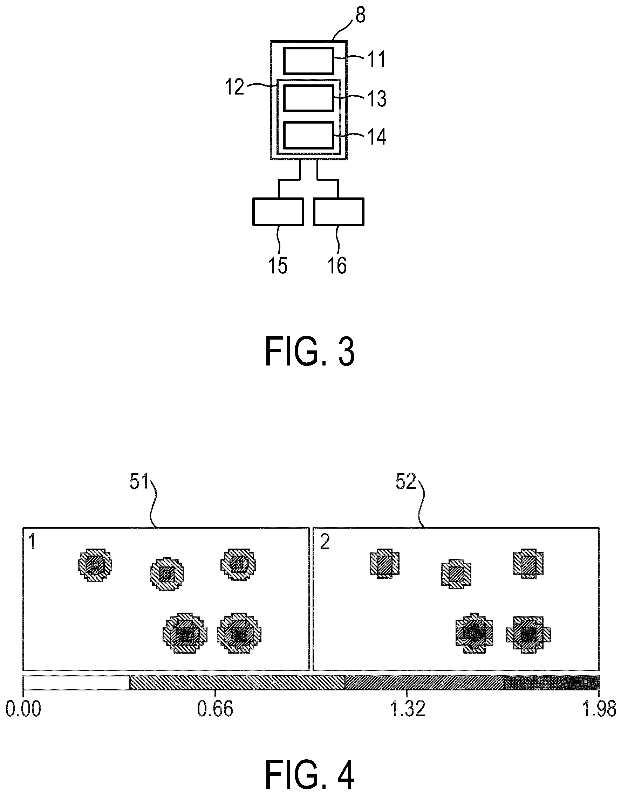

The radiation therapy planning system 8 is schematically and exemplarily shown in Fig. 3. It comprises a quality reducing unit 11 configured to reduce the quality of a higher quality ODM, comprising bixels, which provide target beamlet fluences, to create a quality reduced ODM, and a treatment plan generating unit 12 configured to generate a treatment plan by performing an optimization that includes a first optimization that independently optimizes for each leaf-pair 41 of the MLC 4 one or more leaf configurations such that the cumulative fluence of the radiation beam 3 shaped by the one or more leaf configurations of the leaf-pair 41 approximates a corresponding portion of the quality reduced ODM. The treatment plan generating unit 12 is further configured to perform a higher quality optimization procedure on the higher quality ODM based on a local search algorithm that is initialized with the generated treatment plan in order to generate a higher quality treatment plan.

According to this embodiment, a global solution to an approximation of the treatment planning problem may be found in a deterministic amount of computation time and with a deterministic amount of memory use. This approximate solution, i.e., the generated treatment plan, can then be used to initialize a local search algorithm for a more accurate problem formulation that better captures all machine constraints in order to generate a higher quality treatment plan for the higher quality ODM. Thus, the invention replaces a critical step in the traditional intensity modulated radiation therapy (IMRT) or VMAT planning process, which is currently treated heuristically or with algorithms without convergence guarantees, with a deterministic, global algorithm.

This embodiment assumes that one or more ODMs (also known as "fluence maps" or "intensity maps") for a treatment plan have already been found using a fluence map optimization (FMO) technique. Since the FMO problem can be addressed with convex optimization techniques time (see J. Unkelbach et al. (2015), ibid), a global solution to the FMO problem can be found using gradient-based local search approaches.

In this embodiment, the quality reducing unit 11 is configured to reduce the higher quality ODM 51 in spatial resolution and/or in the number of different possible beamlet fluence values. This is illustrated in Fig. 4, which shows schematically and exemplarily a higher quality ODM 51 and a quality reduced ODM 52. As can be seen, in this example the higher quality ODM 51 is reduced both in spatial resolution and in the number of different possible beamlet fluence values to create the quality reduced ODM 52. This may be achieved, for instance, through a combination of a resampling processing that reduces the spatial resolution and a quantization processing that reduces the number of different possible beamlet fluence values.

With returning reference to Fig. 2, the treatment plan generating unit 12 comprises a DP unit 13 configured to perform the optimization for each leaf-pair 41 of the MLC 4 using a DP forward recursion.

The number of the one or more leaf configurations for a given leaf-pair may typically not be known beforehand. The DP forward recursion may thus be run, for instance, until the cumulative fluence of the radiation beam 3 shaped by the one or more leaf configurations of the leaf-pair 41 perfectly corresponds to the corresponding portion of the quality reduced ODM 51 or until a desired error criterion is met.

The state space of the DP forward recursion is given by the number of possible cumulative beamlet fluence values at each step of the DP forward recursion, wherein the DP unit 13 is configured to cap the state space for each bixel to the target beamlet fluence for the bixel.

As described above, by capping the state space for each bixel in the quality reduced ODM 52 at the target fluence, the size of the state space can be vastly decreased. Since the fluence contribution from each step is always positive, cost contributions in excess of the target fluence can be handled as a stage cost in the DP formulation, without affecting the optimality of the solution. This idea to cap the state space, in combination with a highly optimized implementation in parallelized integer arithmetic, allows solutions to realistic sized ODMs to be calculated in fractions of a second.

In this embodiment, the treatment plan generating unit 12 comprises a same number determining unit 14 configured to determine a same number of the one or more leaf configurations to be used for each leaf-pair 41 of the MLC 4 to shape the radiation beam 3 such that the cumulative fluence of the shaped radiation beam 3 approximates the quality reduced ODM 52 based on the independently optimized one or more leaf configurations for each leaf pair 41 of the MLC 4.

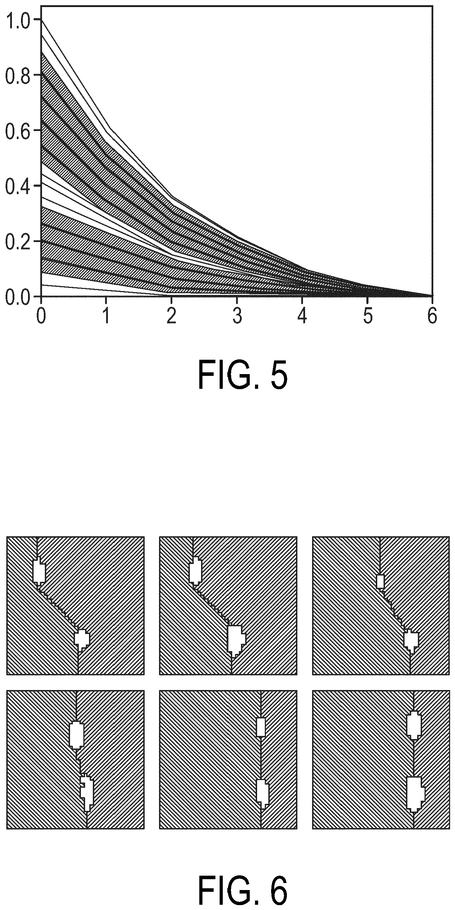

By performing the DP forward recursion for each leaf-pair 41 independently and stopping after each additional leaf configuration to evaluate the result so far, it is possible for each leaf-pair 41 to obtain a trade-off curve between the number of the one or more leaf configurations and the deviation from the target fluence. Such trade-off curves are illustrated in Fig. 5. As can be seen from the figure, for some leaf-pairs 41 a total of 6 leaf configurations are required to perfectly reconstruct the corresponding portion of the quality reduced ODM 52 while for other leaf-pairs 41 only 3, 4 or 5 or even a smaller number of leaf configurations may be necessary. Based on these trade-off curves the same number of the one or more leaf configurations to be used for each leaf-pair 41 of the MLC 4 may be determined such that the apertures of the MLC 4 formed by the determined same number of the one or more leaf configurations of each leaf-pair 41 of the MLC 4 may allow for a perfect reconstruction of the quality reduced ODM 52 or for a reconstruction of the quality reduced ODM 52 such that a desired error criterion is met. For instance, in the illustrated case the same number of the one or more leaf configurations may be determined to be 6. Alternatively, a smaller number may be determined as the same number of the one or more leaf configurations, for instance, 5, which could correspond to the 5 best leaf configurations (which must not necessarily be a subset of the 6 leaf configurations needed for a perfect fit).

With returning reference to Fig. 2, the optimization includes a second optimization that optimizes for each leaf-pair 41 of the MLC 4 the determined same number of the one or more leaf configurations, wherein a regularization is used such that a deviation of the leaf configurations of a currently optimized leaf-pair from the leaf configurations of a neighboring, previously optimized leaf-pair is reduced.

The first optimization generally does not have a unique solution. For instance, other solutions with the same fitting error may be obtained by permutating the order of the leaf configurations, or by exploiting the fact that most leaf-pairs may require less than the determined same number of the one or more leaf configurations to reconstruct the corresponding portion of the reduced quality ODM 52. These degrees of freedom are exploited in this embodiment in the second optimization, e.g., in a second application of the DP algorithm, this time adding a secondary objective to the optimization formulation (DP formulation) that reduces the deviation between neighboring, optimized leaf-pairs 41 without sacrificing the fitting objective.

In this embodiment, the second optimization includes an ordering heuristic such that the optimizing starts with a leaf-pair 41 of the MLC 4 that required a larger number of leaf configurations in the first optimization. Moreover, the second optimization includes a centering of closed leaf configurations such as to smooth the transitions between neighboring leaf-pairs 41 of the MLC 4.

The ordering heuristic, here, is determined based on the trade-off curves between the number of the one or more leaf configurations and the deviation from the target fluence (see Fig. 5) and may employ a greedy strategy. While the use of the ordering heuristic and the centering of the closed leaf configurations may not guarantee optimality in terms of the apertures of the MLC 4, it attempts to get apertures that are reasonable smooth and, thus, consistent with treatment plan quality objectives.

Fig. 6 shows schematically and exemplarily 6 optimized apertures of the MLC 4. As can be seen from the figure, the use of the ordering heuristic and the centering of the closed leaf configurations results in reasonably smooth apertures with a smooth transition between the two openings of the apertures.

In this embodiment, the radiation therapy procedure is a VMAT procedure, wherein each of the apertures of the MLC 4 formed by the determined same number of the one or more leaf configurations of each leaf-pair 41 of the MLC 4 is to be used to shape the radiation beam 3 emitted by the radiation source 2 from a different position on a segment of the arc. For example, the segment of the arc may span an angle of 24 degrees and the different positions from which the radiation source 2 emits the radiation beam may include positions at each 4 degrees or at each 2 degrees of the arc. Since in a VMAT procedure the radiation beam 3 is used in a continuous irradiation mode, these positions may be chosen as control points, wherein the aperture of the MLC between these control points might be modeled by using piecewise linear or piecewise constant models (see below).

In this embodiment, the treatment plan generating unit 12 is configured to perform the optimization for each of two or more segments of the arc, wherein a separate ODM 51, 52 is used for each segment, wherein a consistency constrained is used for consecutive segments such as to smooth the transition between the apertures of the MLC 4 between the consecutive segments.

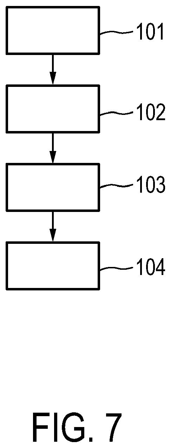

In the following, an embodiment of a radiation therapy planning method for planning a radiation therapy procedure will exemplarily be described with reference to a flowchart shown in Fig. 7. The method may be performed, for instance, by the radiation therapy planning system 8 of Fig. 3.

In step 101, the quality of a higher quality opening density matrix 51, ODM, comprising bixels, which provide target beamlet fluences, is reduced to create a quality reduced ODM 52.

In step 102, a treatment plan is generated by performing an optimization that includes a first optimization that independently optimizes for each leaf-pair 41 of the MLC 4 one or more leaf configurations such that the cumulative fluence of the radiation beam 3 shaped by the one or more leaf configurations of the leaf-pair 41 approximates a corresponding portion of the quality reduced ODM 52.

In step 103, a higher quality optimization procedure is performed on the higher quality ODM 51 based on a local search algorithm that is initialized with the generated treatment plan in order to generate a higher quality treatment plan. The generated treatment plan can then be provided to the controller 8 of the radiation therapy system 1, which controls the radiation therapy system 1 in accordance with the generated treatment plan.

The treatment parameters, which are optimized, i.e. determined, during the generation of the treatment plan, include preferentially the fluence of the radiation beam 3 and the positions of the leafs of the MLC 4 (leaf configurations) for shaping the radiation beam 3. Further treatment parameters can be, for instance, the rotation speed of the gantry 5 and/or the energy of the radiation beam 3. The treatment parameters can be dynamically modulated while the radiation beam 3 continuously irradiates the subject 4.

The trajectory, along which the radiation source 2 is rotated around the subject 4, is called an "arc", wherein during this movement along the arc the radiation source including the MLC will be controlled according to the determined treatment parameters, wherein this control in accordance with the treatment parameters can lead to a change of, for instance, the configuration of the radiation source 2 in quick succession, wherein the treatment parameters and hence possible changes in configuration are determined for several positions along the arc, which can be called "control points". Correspondingly, the radiation therapy planning system 7, particularly the treatment plan generating unit 12, is preferentially configured to determine treatment parameters for each control point along the arc.

The radiation therapy planning system 7 can be configured to provide, for instance, a graphical user interface allowing a user via an input unit 15 like a keyboard, a computer mouse, a touchpad, et cetera and a display 16 to define one or several arcs, i.e., one or several trajectories along which the radiation source 2 is continuously rotated around the subject 5, wherein each arc is described by a discrete set of control points. The treatment parameters are determined for these control points, while for the transitions between the control points the treatment parameters might be modeled by using piecewise linear or piecewise constant models.

The desired dose distribution can simply be, for instance, that only a target like a tumor should receive a therapeutic dose, i.e., a dose being larger than a predefined dose threshold. A corresponding deviation from this desired dose distribution could be defined by the so called "conformity index". The desired dose distribution can also define, for instance, a first dose which should be received by a first percentage of the target and a second dose which should be received by a second percentage of the target, wherein this desired dose distribution could be defined by a homogeneity index, wherein the treatment parameters can be optimized such that the desired homogeneity index is achieved.

The generated treatment plan is preferentially a VMAT treatment plan. VMAT treatment plans have the advantage that they are relatively fast to deliver as the radiation beam is used in a continuous irradiation mode. The VMAT treatment plan can also lead to a very high conformity with the desired dose distribution, especially in comparison to step-and-shoot treatment plans, because the beam energy is smeared out across a larger angular range. For this reason, the respective angular range can be relatively long, i.e., for instance, larger than 180 degrees, and can even be 360 degrees.

The treatment parameters, which can be modulated during radiation delivery, can include, as mentioned above, leaf positions of the MLC, the dose rate, i.e., the fluence of the radiation beam, the gantry speed, the energy of the radiation beam, et cetera. The treatment parameters can also include positions of jaws of the radiation source 2. In particular, the radiation source 2 comprises a linear accelerator for generating the initial radiation beam, i.e., for generating the radiation beam before collimation, wherein in addition to the MLC, the radiation source 2 comprises a further collimation system comprising several jaws, wherein each jaw can block a side of the initial radiation beam. Each jaw can be moved in or out, in order to block a larger or smaller portion at the respective side of the radiation beam. Preferentially, the radiation source 2 has four jaws for blocking a portion of the radiation beam 3 on the left, on the right, on the top and at the bottom. In this way the "field" of the radiation beam 3 can be delimited. This field is then further collimated with the more complex structure of the leafs of the MLC 4.

Although in the above described embodiments the planning system is adapted to plan a VMAT procedure and the radiation therapy system is a VMAT system, which refers to a continuous delivery of radiation along one or several planar arcs, wherein each planar arc covers 360 degrees or less, the planning system can also be used to plan a radiation therapy to be carried out by another radiation therapy system using radiation beams for treating a subject. For instance, instead of only planar trajectories, also arbitrary trajectories on a sphere in three-dimensional space can be considered. In this case instead of planar angles spatial angles on the sphere are to be used. For instance, in an embodiment the subject can be moved, i.e., a patient table can be moved, while the radiation source is rotated around the subject, wherein this movement can be, for instance, a tilting or shifting of the patient table. In this case the trajectory, along which the radiation source is moved relative to the subject, is non-planar. However, also in this case an arc is present being defined as the trajectory along which the radiation source is moved relative to the subject.

The radiation therapy planning system can also be used for planning a radiation therapy procedure for a step-and-shoot intensity modulated radiation therapy (IMRT procedure), especially if the number of positions from which the shaped radiation beam is applied to the subject is relatively large, i.e., for instance, 15 or larger. An example of such an IMRT system, for which the planning system can generate a treatment plan, is disclosed in the article "Bridging the gap between IMRT and VMAT: Dense angular sampled and sparse intensity modulated radiation therapy (DASSIM-RT)" by R. Li et al., Medical Physics, Vol. 38, pages 4912 to 4919 (2001), which is herewith incorporated by reference. In this case, each single position from which the shaped radiation beam is applied is considered to be on one arc and the treatment plan is generated such that corresponding treatment parameters are generated for each arc. The apertures of the MLC formed by the determined same number of the one or more leaf configurations of each leaf-pair of the MLC for each arc are then to be used to sequentially shape the radiation beam emitted by the radiation source from the single position on the arc.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the reduction of the quality of the higher quality ODM, the generation of the treatment plan, et cetera, performed by one or several units or devices can also be performed by any other number of units or devices. These procedures and/or the control of the radiation therapy planning system in accordance with the radiation therapy planning method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a radiation therapy planning system for planning a radiation therapy procedure, wherein the radiation therapy procedure includes rotating a radiation source, which is configured to emit a radiation beam for treating a subject, and an MLC, which comprises multiple leaf-pairs of movable leaves for forming an aperture of the MLC such that the radiation beam is shaped by the aperture, along an arc around the subject in order to apply the shaped radiation beam to the subject from at least one position on the arc. The radiation therapy planning system comprises a quality reducing unit configured to reduce the quality of a higher quality opening density matrix (ODM) comprising bixels, which provide target beamlet fluences, to create a quality reduced ODM, and a treatment plan generating unit configured to generate a treatment plan by performing an optimization that includes a first optimization that independently optimizes for each leaf-pair of the MLC one or more leaf configurations such that the cumulative fluence of the radiation beam shaped by the one or more leaf configurations of the leaf-pair approximates a corresponding portion of the quality reduced ODM. The treatment plan generating unit is further configured to perform a higher quality optimization procedure on the higher quality ODM based on a local search algorithm that is initialized with the generated treatment plan in order to generate a higher quality treatment plan.

## Claims

1. A radiation therapy planning system (8) for planning a radiation therapy procedure, wherein the radiation therapy procedure includes rotating a radiation source (2), which is configured to emit a radiation beam (3) for treating a subject (5), and a multi-leaf collimator (4), MLC, which comprises multiple leaf-pairs (41) of movable leaves for forming an aperture of the MLC (4) such that the radiation beam (3) is shaped by the aperture, along an arc around the subject (5) in order to apply the shaped radiation beam (3) to the subject (5) from at least one position on the arc, wherein the radiation therapy planning system (8) comprises:
- a quality reducing unit (11) configured to reduce the quality of a higher quality opening density matrix (51), ODM, comprising bixels, which provide target beamlet fluences, to create a quality reduced ODM (52), and
- a treatment plan generating unit (12) configured to generate a treatment plan by performing an optimization that includes a first optimization that independently optimizes for each leaf-pair (41) of the MLC (4) one or more leaf configurations such that the cumulative fluence of the radiation beam (3) shaped by the one or more leaf configurations of the leaf-pair (41) approximates a corresponding portion of the quality reduced ODM (52),
wherein the treatment plan generating unit (12) is further configured to perform a higher quality optimization procedure on the higher quality ODM (51) based on a local search algorithm that is initialized with the generated treatment plan in order to generate a higher quality treatment plan.

2. The radiation therapy planning system (8) as defined in claim 1, wherein the quality reducing unit (11) is configured to reduce the higher quality ODM (51) in spatial resolution and/or in the number of different possible beamlet fluence values.

3. The radiation therapy planning system (8) as defined in claim 1, wherein the treatment plan generating unit (12) comprises a dynamic programming, DP, unit (13) configured to perform the optimization for each leaf-pair (41) of the MLC (4) using a DP forward recursion.

4. The radiation therapy planning system (8) as defined in claim 3, wherein the state space of the DP forward recursion is given by the number of possible cumulative beamlet fluence values at each step of the DP forward recursion, wherein the DP unit (13) is configured to cap the state space for each bixel to the target beamlet fluence for the bixel.

5. The radiation therapy planning system (8) as defined in claim 1, wherein the treatment plan generating unit (12) comprises a same number determining unit (14) configured to determine a same number of the one or more leaf configurations to be used for each leaf-pair (41) of the MLC (4) to shape the radiation beam (3) such that the cumulative fluence of the shaped radiation beam (3) approximates the quality reduced ODM (52) based on the independently optimized one or more leaf configurations for each leaf pair (41) of the MLC (4).

6. The radiation therapy planning system (8) as defined in claim 5, wherein the optimization includes a second optimization that optimizes for each leaf-pair (41) of the MLC (4) the determined same number of the one or more leaf configurations, wherein a regularization is used such that a deviation of the leaf configurations of a currently optimized leaf-pair from the leaf configurations of a neighboring, previously optimized leaf-pair is reduced.

7. The radiation therapy planning system (8) as defined in claim 6, wherein the second optimization includes an ordering heuristic such that the optimizing starts with a leaf-pair (41) of the MLC (4) that required a larger number of leaf configurations in the first optimization.

8. The radiation therapy planning system (8) as defined in claim 6, wherein the second optimization includes a centering of closed leaf configurations such as to smooth the transitions between neighboring leaf-pairs (41) of the MLC (4).

9. The radiation therapy planning system (8) as defined in claim 6, wherein the radiation therapy procedure is a step-and-shot intensity modulated radiation therapy, IMRT, procedure, wherein the apertures of the MLC (4) formed by the determined same number of the one or more leaf configurations of each leaf-pair (41) of the MLC (4) are to be used to sequentially shape the radiation beam (3) emitted by the radiation source (2) from a single position on the arc.

10. The radiation therapy planning system (8) as defined in claim 1, wherein the radiation therapy procedure is a volumetric modulated arc therapy, VMAT, procedure, wherein each of the apertures of the MLC (4) formed by the determined same number of the one or more leaf configurations of each leaf-pair (41) of the MLC (4) is to be used to shape the radiation beam (3) emitted by the radiation source (2) from a different position on a segment of the arc.

11. The radiation therapy planning system (8) as defined in claim 10, wherein the treatment plan generating unit (12) is configured to perform the optimization for each of two or more segments of the arc, wherein a separate ODM (51, 52) is used for each segment, wherein a consistency constrained is used for consecutive segments such as to smooth the transition between the apertures of the MLC (4) between the consecutive segments.

12. A radiation therapy system (1), comprising:
- a radiation source (2) configured to emit a radiation beam (3) for treating a subject (5),
- a multi-leaf collimator (4), MLC, comprising multiple leaf-pairs (41) of movable leaves for forming an aperture of the MLC (4) such that the radiation beam (3) is shaped by the aperture,
- a rotating unit (6) for rotating the radiation source (2) and the MLC (4) along an arc around the subject (5) in order to apply the shaped radiation beam (3) to the subject (5) from at least one position on the arc,
- a radiation therapy planning system (8) for planning a radiation therapy procedure as defined in claim 1,
- a controller (9) for controlling the radiation source (2), the MLC (4) and the rotating unit (6) in accordance with the planned radiation therapy procedure.

13. A radiation therapy planning method for planning a radiation therapy procedure, wherein the radiation therapy procedure includes rotating a radiation source (2), which is configured to emit a radiation beam (3) for treating a subject (5), and a multi-leaf collimator (4), MLC, which comprises multiple leaf-pairs (41) of movable leaves for forming an aperture of the MLC (4) such that the radiation beam (3) is shaped by the aperture, along an arc around the subject (5) in order to apply the shaped radiation beam (3) to the subject (5) from at least one position on the arc, wherein the radiation therapy planning method comprises:
- reducing, by a quality reducing unit (11), the quality of a higher quality opening density matrix (51), ODM, comprising bixels, which provide target beamlet fluences, to create a quality reduced ODM (52),
- generating, by a treatment plan generating unit (12), a treatment plan by performing an optimization that includes a first optimization that independently optimizes for each leaf-pair (41) of the MLC (4) one or more leaf configurations such that the cumulative fluence of the radiation beam (3) shaped by the one or more leaf configurations of the leaf-pair (41) approximates a corresponding portion of the quality reduced ODM (52), and
- performing, by the treatment plan generating unit (12), a higher quality optimization procedure on the higher quality ODM (51) based on a local search algorithm that is initialized with the generated treatment plan in order to generate a higher quality treatment plan.

14. A radiation therapy planning computer program for planning a radiation therapy procedure, the computer program comprising program code means for causing a radiation therapy planning system (8) as defined in claim 1 to carry out the steps of the radiation therapy planning method as defined in claim 13, when the computer program is run on a computer controlling the radiation therapy planning system (8).
